# EUROPEAN PATENT APPLICATION

(11) **EP 4 692 303 A1**
(43) Date of publication of application: **11.02.2026**
(21) Application number: 24780073.3
(22) Date of filing: 22.03.2024
(51) Int. Cl.: C12M 1/34, C12M 1/00, C12N 15/09, C12Q 1/6806, C12Q 1/6837, C12Q 1/6876

(54) **NUCLEIC ACID DETECTION CHIP**

(30) Priority: 24.03.2023 JP 2023047707
(71) Applicant: NATIONAL UNIVERSITY CORPORATION TOKAI NATIONAL HIGHER EDUCATION AND RESEARCH SYSTEM, Nagoya-shi, Aichi 464-8601 (JP)
(72) Inventor: NOTAGUCHI, Michitaka, Nagoya-shi, Aichi 464-8601 (JP); HARA, Mitsuo, Nagoya-shi, Aichi 464-8601 (JP); KAWAKATSU, Yaichi, Nagoya-shi, Aichi 464-8601 (JP); ARIMA, Akihide, Nagoya-shi, Aichi 464-8601 (JP)
(74) Representative: Hoffmann Eitle
(86) International application number: PCT/JP2024/011489
(87) International publication number: WO 2024/203955

(57) **Abstract**

Provided is a chip that enables a nucleic acid in a biological sample (in particular, a plant sample) to be detected with higher sensitivity.

A nucleic acid detection chip includes an inorganic substrate and a nucleic acid probe, (x) the nucleic acid probe being linked via an amino group linked to the inorganic substrate and/or (y) the nucleic acid probe being linked by an amide bond between an amino group linked to the inorganic substrate and the nucleic acid probe that has an active ester group or a carboxy group.

## Description

### Technical Field

The present invention relates to a nucleic acid detection chip and the like.

### Background Art

Plant growth is inhibited by environmental stresses, such as nutrient deficiency and microbial infection. Further, during the growth process, plants undergo phenomena that are important for the harvest of crops, such as the growth and thickening of stems and trunks, flowering and fruition, the growth of fruits, and the growth of root portions, such as tubers and tuberous roots.

Patent Literature (PTL) 1 discloses a chip that can be used to determine the state of a plant, such as the presence or absence of abiotic stress (e.g., environmental stress), biotic stress (e.g., plant virus infection), and the prospects for plant development, growth, and physiological phenomena, such as flowering, that enables the determination to be more simply and efficiently performed, that can be used while cultivating crops, if necessary, and that enables the state to be determined on the spot at a cultivation site, if necessary. This technique makes it possible to simply detect a nucleic acid, such as miRNA or mRNA, in, for example, a plant sample.

### Citation List

### Patent Literature

PTL 1: JP2021-065112A

### Summary of Invention

### Technical Problem

An object of the present invention is to provide a chip that enables a nucleic acid in a biological sample (in particular, a plant sample) to be detected with higher sensitivity.

### Solution to Problem

The present inventors conducted extensive research in light of the background art and the object described above and found that the object can be achieved by a nucleic acid detection chip comprising an inorganic substrate and a nucleic acid probe, (x) the nucleic acid probe being linked via an amino group linked to the inorganic substrate and/or (y) the nucleic acid probe being linked by an amide bond between an amino group linked to the inorganic substrate and the nucleic acid probe that has an active ester group or a carboxy group. The inventors conducted further research based on this finding and accomplished the present invention. Specifically, the present invention includes the following embodiments.

Item 1. A nucleic acid detection chip comprising an inorganic substrate and a nucleic acid probe,
(x) the nucleic acid probe being linked via an amino group linked to the inorganic substrate; and/or
(y) the nucleic acid probe being linked by an amide bond between an amino group linked to the inorganic substrate and the nucleic acid probe that has an active ester group or a carboxy group.

Item 2. The nucleic acid detection chip according to Item 1,
wherein
(x1) a group represented by formula (x1): 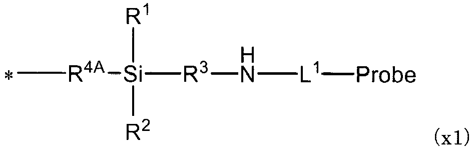
   wherein R¹ and R² are the same or different, and each represents an alkyl group, a hydrogen atom, an alkoxy group, a halogen atom, a site of attachment to a silane coupling agent, or a site of attachment to the inorganic substrate; R³ represents a linker; R^{4A} represents an oxygen atom or a single bond; L¹ represents a linker; Probe represents a nucleic acid probe; and * represents a site of attachment to the inorganic substrate,
   is attached to the inorganic substrate; and/or
(y1) a group represented by formula (y1): 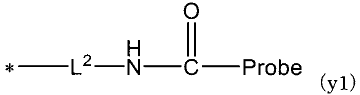
   wherein L² represents a linker; Probe represents a nucleic acid probe; and * represents a site of attachment to the inorganic substrate,
   is attached to the inorganic substrate.

Item 3. The nucleic acid detection chip according to Item 1 or 2, wherein (xy) the nucleic acid probe is linked by an amide bond between an amino group linked to the inorganic substrate and the nucleic acid probe that has an active ester group or a carboxy group.

Item 4. The nucleic acid detection chip according to Item 3,
wherein (xy1) a group represented by formula (xy1): 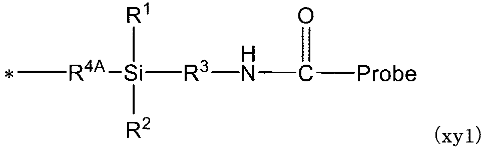
wherein R¹ and R² are the same or different, and each represents an alkyl group, a hydrogen atom, an alkoxy group, a halogen atom, a site of attachment to a silane coupling agent, or a site of attachment to the inorganic substrate; R³ represents a linker; R^{4A} represents an oxygen atom or a single bond; Probe represents a nucleic acid probe; and * represents a site of attachment to the inorganic substrate,
is attached to the inorganic substrate.

Item 5. The nucleic acid detection chip according to Item 4, wherein R¹ and R² are the same or different, and each represents an alkyl group.

Item 6. The nucleic acid detection chip according to any one of Items 1 to 5, wherein the nucleic acid probe is an mRNA and/or miRNA detection probe.

Item 7. The nucleic acid detection chip according to any one of Items 1 to 6, comprising:
(A) a sample injection portion; and
(C) a flow channel connected to the sample injection portion A and having a nucleic acid probe immobilization region.

Item 8. The nucleic acid detection chip according to any one of Items 1 to 7, which is for use in detecting a plant nucleic acid.

Item 9. A method for determining a state of a plant, comprising:
(a) bringing a sample of the plant into contact with the nucleic acid detection chip of any one of Items 1 to 8;
(b) detecting a signal on the nucleic acid probe; and
(c) determining the state of the plant using a value of the signal as an index.

Item 10. A method for cultivating a plant, comprising:
(a) bringing a sample of the plant into contact with the nucleic acid detection chip of any one of Items 1 to 8;
(b) detecting a signal on the nucleic acid probe;
(c) determining a state of the plant using a value of the signal as an index; and
(d) adjusting a cultivation condition for the plant based on a result of the determination.

Item 11. A method for producing the nucleic acid detection chip of any one of Items 1 to 8, comprising:
(X) reacting an inorganic substrate with a silane coupling agent; and/or
(Y) reacting an amino group linked to the inorganic substrate with a nucleic acid probe having an active ester group or a carboxy group.

### Advantageous Effects of Invention

The present invention provides a chip that enables a nucleic acid in a biological sample (in particular, a plant sample) to be detected with higher sensitivity, a method for using the chip, and a method for producing the chip.

### Brief Description of Drawings

Fig. 1 shows a reaction scheme for preparing amino group-coated glass in Examples 1 and 2.
Fig. 2 shows a reaction scheme (conventional method) for immobilizing a DNA probe in Example 3.
Fig. 3 shows a reaction scheme (new method) for immobilizing a DNA probe in Example 3.
Fig. 4 shows the results of XPS measurement of the surface of the glass prepared in Example 1.
Fig. 5 shows the results of AFM observation of the surfaces of the glass prepared in Example 1 and commercially available glass.
Fig. 6 shows the results of XPS measurement of the surface of the glass prepared in Example 2.
Fig. 7 shows the results of comparing the detection signal of 1 nM miR399 between commercially available amino group-coated glass and in-house-made amino group-coated glass (Example 3).
Fig. 8 shows the results of a comparison between a conventional method and a method using an NHS ester as a method for immobilizing DNA (Example 3).
Fig. 9 shows the results of a signal amplification experiment using a biotinylated anti-streptavidin antibody, by using a method for immobilizing a DNA probe using in-house-made amino group-coated glass and NHS ester-DNA (Example 4).
Fig. 10 shows the growth states of tomatoes subjected to stress treatment (Example 5).
Fig. 11 shows the results of detecting miR399 in tomatoes using a device (Example 5).
Fig. 12 shows the results of signal detection of tomato samples treated to express miRNAs, using a diagnostic device (Example 6).
Fig. 13 shows the results of examining the expression levels of target miRNAs by qRT-PCR (Example 6).

### Description of Embodiments

In the present specification, the terms "comprising" and "containing" include the concepts of comprising, containing, consisting essentially of, and consisting of.

### 1. Nucleic Acid Detection Chip

In one aspect, the present invention relates to a nucleic acid detection chip comprising an inorganic substrate and a nucleic acid probe, (x) the nucleic acid probe being linked via an amino group linked to the inorganic substrate and/or (y) the nucleic acid probe being linked by an amide bond between an amino group linked to the inorganic substrate and the nucleic acid probe that has an active ester group or a carboxy group (which may also be referred to as "the chip of the present invention" in the present specification). The following describes the nucleic acid detection chip.

The inorganic substrate is not particularly limited as long as it is capable of being linked to a nucleic acid probe and can be used as a nucleic acid detection chip. Examples of materials for the inorganic substrate include glass, metal, and the like, and, for example, glass is preferred from the viewpoint of detection sensitivity and the like. The shape of the inorganic substrate is not particularly limited. The inorganic substrate may take, for example, a plate shape (an inorganic substrate), a spherical shape, or the like.

The nucleic acid probe is not particularly limited as long as it is a nucleic acid that is capable of hybridizing with a nucleic acid to be detected based on complementary base pairing.

In the present specification, "nucleic acid" includes not only DNA and RNA, but also known chemically modified DNA and RNA as described below. To prevent degradation by hydrolases, such as nucleases, the phosphate residue (phosphate) of each nucleotide may be replaced with, for example, a chemically modified phosphate residue, such as phosphorothioate (PS), methylphosphonate, or phosphorodithionate. The hydroxyl group at position 2 of a ribose of each ribonucleotide may be replaced with -OR (R represents, for example, CH₃ (2'-O-Me), CH₂CH₂OCH₃ (2'-O-MOE), CH₂CH₂NHC(NH)NH₂, CH₂CONHCH₃, or CH₂CH₂CN) . Additionally, the base moiety (pyrimidine, purine) may be chemically modified by, for example, introduction of a methyl group or a cationic functional group into position 5 of the pyrimidine base, or replacement of the carbonyl group at position 2 with thiocarbonyl. Additionally, the nucleic acid also includes, but is not limited to, those in which the phosphoric acid moiety or hydroxyl moiety is modified with biotin, an amino group, a lower alkyl amine group, an acetyl group, or the like. Additionally, for example, BNA (LNA) in which the conformation of the sugar moiety is fixed to N-type by crosslinking 2'-oxygen and 4'-carbon in the sugar moiety of the nucleotide can also be used.

The nucleic acid to be detected may preferably be RNA, such as mRNA or miRNA. More specific examples include a group of molecules in organisms that serve as indicators of growth conditions and that are directly encoded by a group of environmental response genes, a group of genes related to the development and growth of organs, tissues, and cells, a group of stress response genes, and the like; mRNAs of the group of environmental response genes and the group of genes related to the development and growth of organs, tissues, and cells; miRNAs that control the expression of these mRNAs; and the like. Even more specific examples include a group of small RNAs, such as miR399 (which responds to phosphate deficiency), miR395 (which responds to sulfate deficiency), miR172 (which is produced during flowering (transition from vegetative growth to reproductive growth)), and miR156 (which is produced during tuber formation); mRNAs of a group of signaling molecules that moves systemically through the phloem and xylem, which are vascular bundles of plants, such as a group of mRNAs that that can move long distances within an individual, mRNAs of FT proteins (which regulate flowering (as a florigen), tuber formation (as a tuberigen), dormant bud growth, etc.), mRNAs of TSF proteins (which are homologous proteins of FT and have properties and functions similar to those of FT), mRNAs of Hd3a proteins (rice FT homologous proteins), mRNAs of RFT1 proteins (rice FT homologous proteins), mRNAs of ZCN8 proteins (maize FT homologous proteins), mRNAs of AFT proteins (which suppress flowering as anti-florigens), mRNAs of HY5 proteins and CLAVATA3/ESR-related (CLE) peptides (which respond to an increase in the degree of symbiosis with rhizobia (nitrogen fixation by rhizobia) in leguminous plants), mRNAs of C-terminally encoded peptide (CEP) peptides (which respond to nitrogen deficiency), and mRNAs of CEP downstream (CEPD) proteins (which respond to nitrogen deficiency); nucleic acids derived from viruses from a pathological diagnostic viewpoint; and the like.

The nucleic acid probe contains a complementary region that hybridizes to the nucleic acid to be detected based on complementary base pairing. The base sequence of the complementary region is complementary to all or part of the nucleic acid to be detected. The base length of the complementary base sequence is, for example, 8 bases or more, and preferably 10 bases or more, and is, for example, 5% or more, preferably 10% or more, more preferably 20% or more, even more preferably 30% or more, and still even more preferably 40% or more, based on the base length of the nucleic acid to be detected taken as 100%.

In the present specification, the term "complementary" includes not only complete base complementarity (completely complementary: e.g., A and T or U, and G and C), but also complementarity to the extent that they can hybridize under stringent conditions. The stringent conditions can be determined based on the melting temperature (Tm) of nucleic acids, as taught by Berger and Kimmel (1987, Guide to Molecular Cloning Techniques Methods in Enzymology, Vol. 152, Academic Press, San Diego CA). Examples of washing conditions after hybridization generally include about "1×SSC, 0.1% SDS, 37°C." It is preferable that the hybridized state is maintained even after washing under such conditions. Although it is not particularly limited, examples of the washing conditions include more stringent hybridization conditions of about "0.5×SSC, 0.1% SDS, 42°C," and even more stringent hybridization conditions of about "0.1×SSC, 0.1% SDS, 65°C." Specifically, the base sequence of the complementary region is a base sequence having an identity of, for example, 85% or more, preferably 90% or more, more preferably 95% or more, even more preferably 98% or more, still even more preferably 99% or more, and particularly preferably 100% to the base sequence completely complementary to all or a part of the nucleic acid to be detected.

In the present specification, the term "identity" of base sequences refers to the degree to which two or more contrastable base sequences match each other. Thus, the higher the degree of match between two base sequences, the higher the identity or similarity of those sequences. The level of base sequence identity is determined, for example, by using FASTA, which is a tool for sequence analysis, with default parameters. Alternatively, the level of base sequence identity can be determined by using the BLAST algorithm by Karlin and Altschul (Karlin S, Altschul SF. "Methods for assessing the statistical significance of molecular sequence features by using general scoringschemes," Proc Natl Acad Sci USA. 87: 2264-2268 (1990); Karlin S, Altschul SF. "Applications and statistics for multiple high-scoring segments in molecular sequences." Proc Natl Acad Sci USA. 90:5873-7 (1993)). A program called "BLASTN" based on this BLAST algorithm has been developed. The specific techniques of these analysis methods are known and can be found on the website of the National Center of Biotechnology Information (NCBI) (http://www.ncbi.nlm.nih.gov/).

The nucleic acid probe preferably has a linker region in addition to the complementary region. The linker region is not particularly limited as long as it is a region that does not significantly inhibit hybridization between the nucleic acid to be detected and the nucleic acid probe, and examples include a nucleic acid linker composed of any base sequence (e.g., poly-T), an alkyl linker, a polymer linker, such as PEG (polyethylene glycol), and the like. The linker region is preferably a nucleic acid linker. When the linker region is a nucleic acid linker, the base length of the linker is, for example, 5 bases or more, preferably 5 to 100 bases, more preferably 5 to 50 bases, even more preferably 5 to 30 bases, and still even more preferably 7 to 20 bases.

In embodiment (x) of the chip of the present invention, the nucleic acid probe is linked via an amino group linked to the inorganic substrate.

The method for linking an amino group to the inorganic substrate is not particularly limited, and preferred examples include a method using a silane coupling agent. When a silane coupling agent having an amino group is used, the amino group can be linked to the inorganic substrate by reacting the inorganic substrate with the silane coupling agent. When a silane coupling agent having a reactive structure (e.g., an Si-H bond, a vinyl group, or the like) is used, an amino group can be linked to the inorganic substrate by reacting the inorganic substrate with the silane coupling agent and then linking a compound having an amino group (e.g., a chain compound) via the reactive structure (for example, by using a hydrosilylation reaction or a thiol-ene reaction).

The silane coupling agent is not particularly limited as long as it has, in the molecule, an amino group (for example, one to three amino groups, preferably one amino group) or a reactive structure (for example, a Si-H bond, a vinyl group, or the like), and a reactive group that can be chemically bonded to the inorganic substrate. Examples of reactive groups that can be chemically bonded to the inorganic substrate include an alkoxy group, a halogen atom, and the like. Among these, an alkoxy group is particularly preferable. The alkoxy group may be linear or branched. From the viewpoint of reactivity, the alkoxy group is preferably a linear alkoxy group having 1 to 4 (in particular, 1 to 2) carbon atoms. Preferred examples of monofunctional silane coupling agents include a compound represented by formula (x2): 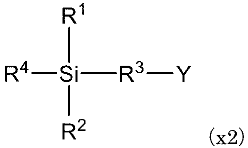 wherein R¹ and R² are the same or different, and each represents an alkyl group, a hydrogen atom, an alkoxy group, or a halogen atom; R³ represents a linker or a single bond; R⁴ represents an alkoxy group or a halogen atom; Y represents an amino group or a vinyl group when R³ is a linker and represents an amino group, a vinyl group, or a hydrogen atom when R³ is a hydrogen atom.

The alkyl group represented by R¹ or R² may be linear or branched, and is preferably a linear alkyl group having 1 to 4 (in particular, 1 to 2) carbon atoms.

The alkoxy group represented by R¹ or R² is the same as the alkoxy group described above.

Examples of the halogen atom represented by R¹ or R² include fluorine, chlorine, bromine, iodine, and the like.

R¹ and R² are each preferably an alkyl group or a hydrogen atom, and particularly preferably an alkyl group. That is, the silane coupling agent is particularly preferably a monofunctional silane coupling agent.

The linker represented by R³ is not particularly limited, and may be, for example, a linear or branched divalent group having 1 to 8 main chain constituent atoms, and preferably an alkylene group or alkenylene group in which a carbon atom constituting the chain may be replaced by a heteroatom (S, O, N, or the like). R³ is particularly preferably a hydrocarbon group.

The hydrocarbon group represented by R³ is a divalent hydrocarbon group, and may be, for example, an alkylene group, an alkenylene group, an arylene group, or a combination thereof. The alkylene group and alkenylene group may be linear or branched, and are preferably a linear alkylene group and linear alkenylene group that have 1 to 8 (more preferably 2 to 6, and particularly preferably 3 to 4) carbon atoms. The arylene group may be, for example, an arylene group having 4 to 12 carbon atoms, such as a phenylene group or a naphthylene group. R³ is preferably an alkylene group.

The alkoxy group represented by R⁴ is the same as the alkoxy group described above.

The halogen atom represented by R⁴ is the same as the halogen atom described above.

R⁴ is preferably an alkoxy group.

Y is particularly preferably an amino group. When Y is an amino group, the chip of the present invention can be produced simply with fewer steps.

In embodiment (x), the silane coupling agent is linked to the inorganic substrate, and the nucleic acid probe is linked via a reaction with an amino group on the silane coupling agent linked to the inorganic substrate or with an amino group linked to the silane coupling agent (and optionally a further separate reaction). In embodiment (x), it is more preferred that (x1) a group represented by formula (x1) is attached to the inorganic substrate: 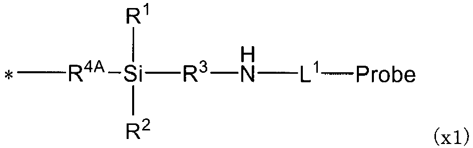 wherein R¹ and R² are the same or different, and each represents an alkyl group, a hydrogen atom, an alkoxy group, a halogen atom, a site of attachment to a silane coupling agent, or a site of attachment to the inorganic substrate; R³ is as defined above; R^{4A} represents an oxygen atom or a single bond; L¹ represents a linker; Probe represents a nucleic acid probe; and * represents a site of attachment to the inorganic substrate.

The alkyl group, alkoxy group, and halogen atom represented by R¹ or R² are the same as those described above.

In the site of attachment to a silane coupling agent represented by R¹ or R², the target silane coupling agent encompasses a free silane coupling agent and a silane coupling agent linked to another substance (for example, the inorganic substrate represented by *).

The linker represented by L¹ is not particularly limited as long as it is a moiety formed by a reaction with the amino group of the monofunctional silane coupling agent linked to the inorganic substrate (and optionally a further separate reaction). The linker may have a linear or branched (preferably linear) chain structure (the one or more main chain constituent atoms may include, for example, carbon, nitrogen, sulfur, and/or oxygen atoms, such as those in an alkylene group or a heteroalkylene group) having, for example, 1 to 50 (from the viewpoint of detection sensitivity and the like, preferably 1 to 20, more preferably 1 to 10, even more preferably 1 to 5, and still even more preferably 1 to 3) carbon atoms in the main chain. The linker may be substituted with an oxo group. The linker may have -C(=O)- in the main chain.

The nucleic acid probe represented by Probe is the same as the nucleic acid probe described above.

The group represented by formula (x1) can be attached to an atom in the inorganic substrate (when the inorganic substrate is glass, a silicon atom constituting the glass).

In embodiment (x), from the viewpoint of detection sensitivity and the like, it is preferred that the surface of the inorganic substrate to which the silane coupling agent is linked, which is the base to which the nucleic acid probe is to be linked, is smoother. The height of aggregates on the surface may be preferably 30 nm or less, more preferably 20 nm or less, and even more preferably 10 nm or less. The height of the aggregates is measured by AFM observation according to the method in Example 1.

In embodiment (y) of the chip of the present invention, the nucleic acid probe is linked by an amide bond between an amino group linked to the inorganic substrate and the nucleic acid probe that has an active ester group or a carboxy group (preferably an active ester group) (preferably at an end or a linker region, particularly preferably at the end on the linker region side).

The amino group linked to the inorganic substrate is an amino group that is linked directly or indirectly (for example, via a linker) to an atom that constitutes the inorganic substrate.

The active ester group is not particularly limited as long as it is capable of forming an amide bond with an amino group, and is, for example, a group in which a carboxyl group (-COOH) is converted to a highly reactive active ester. Examples include active ester groups obtained using N-hydroxysuccinimide (e.g., -C(=O)OSu; Su is succinimide), groups in which a carboxylic acid is converted to mixed acid anhydride (e.g., -C(=O)OC(=O)R; R is a C₁-C₆ alkyl group), imidazolide groups obtained using carbonylimidazole (CDI) (e.g., -C(=O)-Im; Im is a 1-imidazolyl group), and the like.

In embodiment (y), it is more preferred that (y1) a group represented by formula (y1) is attached to the inorganic substrate: 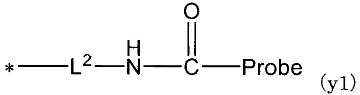 wherein L² represents a linker; Probe and * are as defined above.

The linker represented by L² is not particularly limited as long as it is a moiety that is introduced when linking an amino group onto the inorganic substrate. The linker may have a linear or branched (preferably linear) chain structure (the one or more main chain constituent atoms may include, for example, carbon, silicon, nitrogen, sulfur, and/or oxygen atoms, such as those in an alkylene group or a heteroalkylene group) having, for example, 1 to 50 (from the viewpoint of detection sensitivity and the like, preferably 2 to 20, and more preferably 3 to 10) carbon atoms in the main chain.

The group represented by formula (y1) may be attached to an atom in the inorganic substrate (when the inorganic substrate is glass, a silicon atom constituting the glass).

From the viewpoint of detection sensitivity and the like, the chip of the present invention preferably comprises embodiment (x), and particularly preferably comprises both embodiment (x) and embodiment (y). When the chip of the present invention comprises both, (xy) the nucleic acid probe is linked by an amide bond between an amino group linked to the inorganic substrate and the nucleic acid probe that has an active ester group or a carboxy group. In embodiment (xy), it is more preferred that (xy1) a group represented by formula (xy1) is attached to the inorganic substrate: 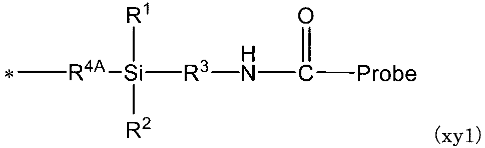 wherein R¹, R², R³, R^{4A}, Probe, and * are as defined above.

The chip of the present invention can be produced by various methods. Preferably, the chip of the present invention can be produced by a method including (X) reacting an inorganic substrate with a silane coupling agent, and/or (Y) reacting an amino group linked to the inorganic substrate with a nucleic acid probe having an active ester group or a carboxy group.

### Step (X)

The inorganic substrate is subjected to surface modification treatment (for example, treatment to introduce a hydroxyl group by plasma treatment) as necessary so that it can react with the silane coupling agent.

The reaction between the inorganic substrate and the silane coupling agent is not particularly limited as long as it is a reaction in which a hydroxyl group exposed on the surface of the inorganic substrate can be reacted with the reactive group (e.g., an alkoxy group) of the silane coupling agent to form a chemical bond (e.g., Si-O-Si). For this reaction, either a gas phase method or a liquid phase method can be used. From the viewpoint of, for example, simplicity and enabling further reduction in the amount of silane coupling agent, a gas phase method is preferable.

Specifically, in the gas phase method, the reaction is performed in a sealed reaction vessel containing, for example, a carrier holding the silane coupling agent, and the inorganic substrate. The silane coupling agent and carrier are not particularly limited as long as the silane coupling agent is volatilizable. For example, 3-aminopropyldimethylmethoxysilane (APDMMS) or 3-aminopropyldimethylmethoxysilane (APDMES) and filter paper can be used. The amount of the silane coupling agent is preferably 0.1 to 2 µL/cm², and more preferably 0.2 to 1 µL/cm², based on the surface area of the inorganic substrate. The reaction conditions may be, for example, as follows: 10 to 50°C for 6 to 24 hours.

Specifically, in the liquid phase method, the reaction is performed in a state in which the inorganic substrate is in contact with (preferably immersed in) a silane coupling agent solution. The concentration of the silane coupling agent in the silane coupling agent solution is preferably 1 to 15 mass%, and more preferably 2 to 10 mass%. The solvent of the silane coupling agent solution can be appropriately selected, and, for example, N,N-dimethylformamide can be used. The silane coupling agent solution preferably contains a base, such as triethylamine, at a concentration of, for example, 0.1 to 1 mass%. The reaction conditions may be, for example, as follows: 60 to 100°C for 6 to 24 hours.

After step (X), washing is preferably performed. The washing method is not particularly limited, and for example, ultrasonic cleansing can be performed in an organic solvent.

When a silane coupling agent that does not have an amino group is used as the silane coupling agent, a reaction for linking an amino group can be further performed after step (X). For example, a hydrosilylation reaction or a thiol-ene reaction can be used as the reaction. More specifically, for example, a reaction in accordance with or based on the following reaction system can be used.

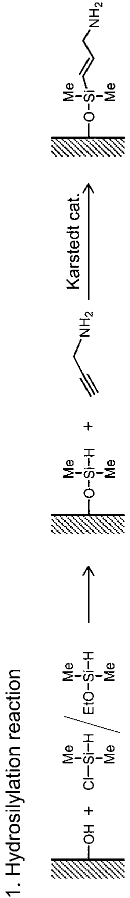

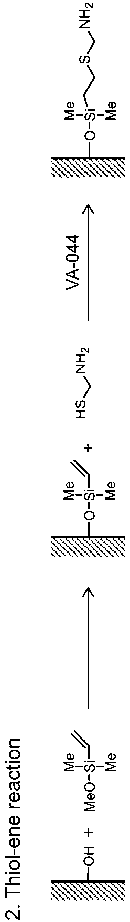

### Step (Y)

The reaction is not particularly limited as long as it is a reaction in which an amino group linked to the inorganic substrate can be reacted with a nucleic acid probe having an active ester group or a carboxy group to form an amide bond. The reaction can be performed by bringing the inorganic substrate to which an amino group is linked into contact with a nucleic acid probe-containing solution.

The concentration of the nucleic acid probe in the nucleic acid probe-containing solution is preferably 0.5 to 10 µM, and more preferably 1 to 5 µM. The solvent of the nucleic acid probe-containing solution is generally water. The nucleic acid probe-containing solution preferably contains a buffer, such as MOPS. The pH of the nucleic acid probe-containing solution is preferably near neutral (for example, 6.0 to 8.0). The nucleic acid probe-containing solution preferably contains a condensing agent (in this case, the concentration of the condensing agent is preferably 10 to 100 mM).

The condensing agent is not particularly limited. Examples include triazine-based condensing agents (e.g., 4-(4,6-dimethoxy-1,3,5-triazin-2-yl)-4-methylmorpholinium chloride (DMT-MM) and (4,6-dimethoxy-1,3,5-triazin-2-yl)-(2-octoxy-2-oxoethyl)dimethylammonium trifluoromethanesulfonate (interfacially assembled DMT-MM)), carbodiimide-based condensing agents (e.g., N,N'-dicyclohexylcarbodiimide (DCC), N,N'-diisopropylcarbodiimide, and 1-ethyl-3-dimethylaminopropyl carbodiimide hydrochloride (EDCI)), uranium-based condensing agents (e.g., 1-[bis(dimethylamino)methylene]-1H-benzotriazolium 3-oxide tetrafluoroborate (TBTU), and 1-[bis(dimethylamino)methylene]-1H-1,2,3-triazolo[4,5-b]pyridinium 3-oxide tetrafluoroborate (TATU)), chloroformate-based condensing agents (e.g., ethyl chloroformate and isobutyl chloroformate), acid halide-based condensing agents (e.g., pivaloyl chloride), imidazole-based condensing agents (e.g., 1,1'-carbonyldiimidazole (CDI)), phosphonium-based condensing agents (e.g., (benzotriazole-1-yloxy)tripyrrolidinophosphonium hexafluorophosphate (PyBOP (registered trademark)), and bromotripyrrolidinophosphonium hexafluorophosphate (PyBrop (registered trademark))), and the like.

After step (Y), washing is preferably performed. The washing method is not particularly limited. For example, a washing operation in which a wash buffer is brought into contact with the resulting product, incubation is performed for a certain period of time, and the wash buffer is removed may be performed.

The reaction scheme in the case of using a nucleic acid probe having an active ester group in step (Y) is shown below. A similar structure is also formed when a nucleic acid probe having a carboxy group is used. In the conventional technique, the active ester group is placed on the substrate side, whereas in step (Y), the active ester group or carboxy group is placed on the nucleic acid probe side, which is a characteristic feature. As a result, a structure in which -C(=O)- and -NH- are arranged in this order from the nucleic acid probe side is formed.

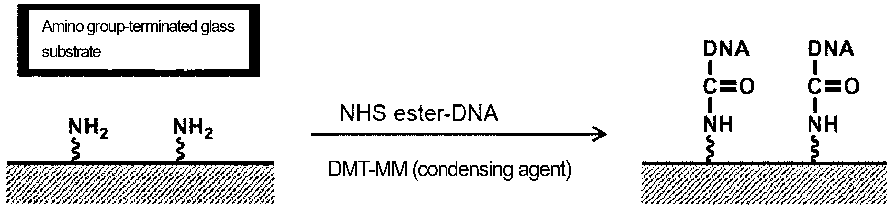

In a preferred embodiment of the present invention, the chip of the present invention is a nucleic acid detection chip in which (xy1) a group represented by formula (xy1) is attached to an inorganic substrate: 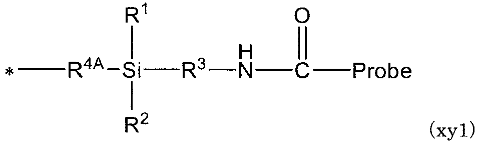 wherein R¹ and R² are the same or different, and each represents an alkyl group or a hydrogen atom; R³ represents a linker; R^{4A} represents an oxygen atom or a single bond; Probe represents a nucleic acid probe; and * represents a site of attachment to the inorganic substrate.

The chip of the present invention is preferably a flow channel chip comprising (A) a sample injection portion and (C) a flow channel connected to the sample injection portion A and having a nucleic acid probe immobilization region.

Polymer resins and the like are suitable for shaping the flow channel for sample delivery, and the flow channel chip of the present invention is preferably composed of their functional components. In particular, polymer resin substrates with high transparency, such as PDMS resin substrates, COC resin substrates, and PMMA resin substrate, are preferable. In addition, inorganic substrates, such as glass and metal, can also be used.

The size of the flow channel chip of the present invention is not particularly limited, and the flow channel chip of the present invention is preferably small in order to be easily and simply handled at a cultivation site or the like. The flow channel chip of the present invention generally has dimensions of 1 to 20 cm in length and 1 to 20 cm in width, and preferably 1 to 5 cm in length and 1 to 5 cm in width. For example, the flow channel chip of the present invention has a size similar to that of a glass slide.

The thickness of the flow channel chip of the present invention can be appropriately set in consideration of the method for shaping the flow channel chip, the depth of the flow channel described below, and the like.

The sample is not particularly limited as long as it is a sample prepared from a living organism, the environment (such as soil, a river, pond, lake, sea, or sewage), or the like and is a sample in which the detection of a substance in the sample is possible by using the sample as is or by purifying the sample as appropriate and using it. An appropriate sample can be used according to the substance to be detected. The sample is particularly preferably a plant sample. Examples of plant samples include ground liquids, extracts, phloem sap, xylem sap, sap, and aqueous liquids from hydathodes of plant tissues such as buds, leaves, stems, flowers, roots, fruits, and seeds.

The sample injection portion A is a portion that is disposed on the flow channel chip of the present invention and that is used for introducing the sample into the flow channel described below. The shape and structure of the sample injection portion A are not particularly limited. The sample injection portion A is generally in the form of a well. The number of sample injection portions A included in the flow channel chip of the present invention may be one or more.

The flow channel chip of the present invention preferably further comprises a sample discharge portion B. The sample discharge portion B is a region that is disposed on the flow channel chip of the present invention and that is used for discharging the sample from the flow channel described below. The shape and structure of the sample discharge portion B are not particularly limited. The sample discharge portion B is generally in the form of a well. The sample discharge portion B may be shaped to discharge a solution to the outside of the flow channel chip, or may be shaped so that a solution is not discharged to the outside of the flow channel chip. When the sample discharge portion B is shaped to discharge a solution to the outside, a structure to insert a tube or the like may be provided, and the solution may be discharged to the outside through the tube or the like. When the sample discharge portion B is shaped so that a solution is not discharged to the outside, for example, an internal space of a certain size can be provided in the sample discharge portion B, and a vacuum or negative pressure can be created in the internal space in advance, whereby the sample solution, buffer solution, or the like from the flow channel C can be discharged into the internal space and then retained within the internal space. In this case, the sample discharge portion B does not necessarily have to be open to the outside. Furthermore, for example, by providing, at a part of the internal space of the sample discharge portion B, a narrow path connected to the outside, and connecting the path to a pump or the like, negative pressure may be created in the internal space in advance or negative pressure may be created during operation of the flow channel chip, whereby a solution from the flow channel C may be discharged into the internal space of the sample discharge portion B and retained in the internal space. Even when a solution is not discharged to the outside of the flow channel chip, the sample discharge portion B may be shaped to be open to the outside. The number of sample discharge portions B included in the flow channel chip of the present invention may be one or more.

The flow channel C is disposed on the flow channel chip of the present invention and is connected to the sample injection portion A. That is, the sample injection portion A and the flow channel C are connected or are disposed in a state in which they can be adjusted to be connected so that a sample introduced from the sample injection portion A passes through the flow channel C. Examples of the latter case include a case in which a flow channel switching portion is provided on the flow channel C, and the flow channel switching portion can switch between a state in which the sample injection portion A and the flow channel C are not connected and a state in which they are connected.

In one embodiment of the flow channel C, the flow channel C connects the sample injection portion A and the sample discharge portion B. That is, the sample injection portion A, the sample discharge portion B, and the flow channel C are connected or are disposed in a state in which they can be adjusted to be connected so that a plant sample introduced from the sample injection portion A can reach the plant sample discharge portion B through the flow channel C. Examples of the latter case include a case in which a flow channel switching portion is provided on the flow channel C, and the flow channel switching portion can switch between a state in which the sample injection portion A, the sample discharge portion B, and the flow channel C are not connected and a state in which they are connected.

The number of flow channels C included in the flow channel chip of the present invention may be one or more.

The flow channel C may be present in various forms on the chip. For example, a groove formed on the surface of the chip can be used as a flow channel. Furthermore, a conduit-like flow channel formed inside the chip can also be used as a flow channel.

The shape of the flow channel C is not particularly limited. The cross-sectional shape of the plane perpendicular to the flow of a sample in the flow channel may be, for example, circular, semicircular (with the chord on top), quadrangular (with one side on top), triangular (with one side on top), or the like. Considering the ease of producing and processing the flow channel, the ease of producing the detection region of the flow channel, and the like, a semicircular or quadrangular shape is preferable.

The shape of the flow channel axis of the flow channel C is not particularly limited as long as the flow of a sample is not impeded when the sample is flowed through the flow channel. The shape of the flow channel axis of the flow channel C may be, for example, a linear shape or a curved shape, and a linear shape is preferable. This is because the flow of the sample is least likely to be impeded. The flow channel axis means the axis in the direction of flow of a fluid (sample) in the flow channel. The flow channel axis may be split into multiple branches from the sample injection portion A, a detection site may be provided at the end of each branch, and detection can be performed in parallel at multiple locations. By repeating the detection reaction multiple times in this manner, highly reproducible results can be obtained.

The size of the flow channel C is not particularly limited, and the flow channel C generally has a width of several micrometers or more and several thousand micrometers or less and a depth of several micrometers or more and several thousand micrometers or less. The flow channel C is preferably a micro-flow channel. The width and height of the flow channel C can be appropriately set within the above ranges depending on the purpose etc. The width of the flow channel is, for example, 10 to 2000 µm, preferably 100 to 1000 µm, more preferably 300 to 700 µm, and even more preferably 400 to 600 µm. The height of the flow channel is 1 to 200 µm, 5 to 100 µm, 10 to 50 µm, or 15 to 25 µm.

The flow channel C has a nucleic acid probe immobilization region thereon. In this region, the nucleic acid probe is attached to the inner wall of the flow channel. The manner in which the nucleic acid probe is attached is embodiment (x) and/or embodiment (y) described above. The number of nucleic acid probe immobilization regions included in one flow channel C may be one or more. When immobilizing the probe, the probe may be immobilized in a limited specific region using a probe immobilization device, and an alignment mark may be provided on the device as an index to identify the probe immobilization position. The number of immobilized nucleic acid probes included in one flow channel C may be one or more.

In one embodiment of the present invention, the flow channel chip of the present invention preferably includes a nucleic acid probe non-immobilization region on the flow channel C and/or a flow channel D having a nucleic acid probe non-immobilization region. When there are a plurality of flow channels C, the flow channel chip preferably includes flow channels D corresponding to the respective flow channels C. Furthermore, it is preferred that the flow channels D and C are designed so that the same plant sample passes through them. Specifically, it is preferred that, for example, both the flow channel C and the flow channel D are connected from one sample injection portion A.

When RNA is the target for detection (in particular, when a plant sample is used), if the degree of purification of the sample is low (for example, when a ground liquid or the like of plant tissue is used), the RNA detection sensitivity and detection accuracy will decrease due to the influence of RNases etc. In this case, by using a plant sample obtained by filtering through a filter (in particular, a protein removal filter), the RNA detection sensitivity and detection accuracy can be greatly improved in a simple manner. The MWCO of the filter is preferably 5 to 100 K, more preferably 5 to 70 K, even more preferably 10 to 50 K, still even more preferably 15 to 40 K, and particularly preferably 20 to 35 K. The filter may be disposed on the flow channel chip of the present invention as described below. The passage through the filter can be carried out by suction or centrifugation, if necessary.

The nucleic acid probe immobilized on the flow channel of the flow channel chip of the present invention may be one nucleic acid probe alone or a combination of two or more nucleic acid probes. A plurality of probes may be arranged one-dimensionally or two-dimensionally. From the viewpoint that it is desirable to use carefully selected plant substances so that they can be handled easily and simply at cultivation sites etc., the number of kinds of nucleic acid probes immobilized on the flow channel of the flow channel chip of the present invention is preferably 1 to 200, more preferably 1 to 100, and even more preferably 1 to 20.

As more specific embodiments and production method of the flow channel chip of the present invention, the embodiments of PTL 1 can be used.

### 2. Apparatus

In one aspect, the present invention relates to an apparatus comprising the chip of the present invention (preferably the flow channel chip of the present invention) (which may also be referred to as "the apparatus of the present invention" in the present specification). The following describes the apparatus.

The apparatus of the present invention preferably includes, in addition to the chip of the present invention, one or more various devices, parts, etc. used for sample detection. Examples of devices include a reservoir for a liquid (e.g., a buffer solution or a washing liquid), a tube for moving a liquid from a reservoir to the flow channel chip of the present invention or the like, a vacuum pump, an imaging portion, a plant sample collection portion, a plant sample grinding portion, a plant sample preparation portion, a plant sample purification portion, a temperature regulator, a voltage and current regulator, a magnetic force regulator, and the like. Other examples include a device having some of the above functions (a reservoir for a liquid (e.g., a buffer solution or a washing liquid), a tube for moving a liquid from a reservoir to the flow channel chip of the present invention or the like, a vacuum pump, a plant sample collection portion, a plant sample preparation portion, and a plant sample purification portion.

The sample purification portion is not particularly limited as long as it is a portion capable of purifying a sample, and can be, for example, a portion where a chromatography carrier is disposed. Furthermore, from the viewpoint of easily improving RNA detection sensitivity and detection accuracy, a plant sample purification portion composed on the filter described above is preferable.

The apparatus of the present invention can be used as, for example, a sample injection apparatus, a detection apparatus, a plant state determination apparatus, etc. by using it as in the method of the present invention described below ("4. Method" section).

As more specific embodiments and production method of the apparatus of the present invention, the embodiments of PTL 1 can be used.

### 3. Kit

In one aspect, the present invention relates to a kit comprising the chip of the present invention (preferably the flow channel chip of the present invention) and/or the apparatus of the present invention (which may also be referred to as "the kit of the present invention" in the present specification). The following describes the kit.

The kit of the present invention preferably includes one or more various reagents, instruments, etc. used for detecting a sample. Examples of reagents include buffer solutions, washing liquids, instruments for preparing samples, reagents for preparing samples, instruments for purifying samples, reagents for purifying samples, labeled substances (e.g., labeled nucleic acids) that have binding properties (preferably specific binding properties) to target substances, reagents necessary for detecting the labels of the labeled substances, instruments necessary for detecting the labels of the labeled substances, reagents necessary for denaturing unwanted components, instruments necessary for denaturing unwanted components, and the like.

The kit of the present invention can be used as, for example, a plant substance detection kit, a plant state determination kit, etc. by using it as in the method of the present invention described below ("4. Method" section).

### 4. Method

In one aspect, the present invention relates to a method comprising (a) bringing a plant sample into contact with the chip of the present invention (preferably, the nucleic acid probe immobilization region of the flow channel chip of the present invention) and (b) detecting a signal on the nucleic acid probe (which may also be referred to as "the method of the present invention" in the present specification). The following describes the method.

In step a, the manner in which the plant sample is brought into contact with the nucleic acid probe immobilization region is not particularly limited. Typically, the plant sample is introduced into the flow channel C by injecting it from the sample injection portion A and is brought into contact with the nucleic acid probe immobilization region in the flow channel C by free diffusion or by providing a flow velocity, thereby performing the process at a low volume and in a short period of time.

In step a, the plant sample to be brought into contact with the nucleic acid probe immobilization region is preferably a plant sample that has been filtered through a filter (particularly a protein removal filter), from the viewpoint of RNA detection sensitivity and detection accuracy. Filtration through a filter may be performed in the sample purification portion in the flow channel chip of the present invention or may be performed before injection into the flow channel chip of the present invention.

The method for injecting the plant sample into the sample injection portion A is not particularly limited, and the plant sample may be injected using a tool suitable for injecting a small amount of a solution sample, such as a pipette, a micropipette, or a syringe, or a portion formed on a device equivalent to the structure of such a tool.

The method for introducing the injected plant sample into the flow channel C is not particularly limited, and, for example, the injected plant sample is introduced into the flow channel C by manipulating air pressure, capillary action, centrifugal force, voltage, current, osmotic pressure, magnetism, or the like. Examples of specific methods include, when manipulating air pressure, adjusting air pressure using a vacuum pump attached to the flow channel C or the sample discharge portion B or adjusting air pressure in the space including the flow channel C in advance; when using capillary action, adjusting the size of the flow channel C to a size that allows capillary action to occur; when using centrifugal force, centrifuging the flow channel chip of the present invention with a centrifuge; when manipulating voltage, adjusting the voltage between the sample injection portion A and the flow channel C or the sample discharge portion B; when manipulating current, applying current between the sample injection portion A and the flow channel C or the sample discharge portion B; when manipulating osmotic pressure, adjusting the osmotic pressure between the sample injection portion A and the flow channel C or the sample discharge portion B; when manipulating magnetism, adjusting magnetism by making a magnetic object float between the sample injection portion A and the flow channel C or the sample discharge portion B; and the like.

The plant sample that has passed through the flow channel C reaches the sample discharge portion B on the flow channel C (for example, the end portion on the flow channel C on the side opposite to the plant sample injection portion). The plant sample that has reached the sample discharge portion B may, for example, remain there as is, or may be appropriately collected thereafter and, for example, reused or discarded. The collection method is not particularly limited, and the plant sample may be collected using a tool suitable for collecting a small amount of solution sample, such as a pipette, a micropipette, or a syringe. Alternatively, for example, when the sample discharge portion B is shaped to have a certain large internal space, the plant sample delivered through the flow channel C may be discharged into the sample discharge portion B, and then part or all of the sample may remain inside the device.

After step a, the flow channel C, particularly the plant substance detection probe immobilization region, is washed as necessary. Washing is performed, for example, by introducing water, a buffer solution, or the like into the flow channel C in the same manner as described above and discharging it to the outside from the sample discharge portion B or discharging it into the internal space of the sample discharge portion B.

In step b, the manner in which the signal is detected is not particularly limited. For example, when the detection target (plant substance) in the plant sample has been labeled in advance, the signal derived from the label may be detected. When the detection target (plant substance) in the plant sample has not been labeled in advance, after step a, the plant substance on the nucleic acid probe immobilization region may be labeled, and then the signal derived from the label may be detected. Alternatively, the intermolecular interaction between the molecule to be detected and the probe molecule may be detected as a change in the electrical resistance of the reaction region. Labeling can be performed according to a known method depending on the type of label. Labeling may be performed by directly attaching a label to the plant substance or by indirectly attaching a label to the plant substance via another substance.

Examples of labels include fluorescent labels, peroxidase labels, alkaline phosphatase labels, β-galactosidase labels, glucose oxidase labels, urease labels, biotin labels, streptavidin labels, magnetic particle labels, gold/gold colloid labels, radioactive substance labels, quantum dot labels, and the like. Fluorescent substances usable for fluorescent labeling can be appropriately selected from fluorescent compounds sold by reagent companies, such as phycobiliproteins, various fluoresceins, and various cyanine dyes, as well as fluorescent proteins, such as GFP.

The plant substance can be detected by the method of the present invention, which comprises step a and step b described above.

The method of the present invention does not necessarily have to be performed under cooling conditions even in the case of easily degradable detection targets (e.g., RNA). According to the method of the present invention, by using a plant sample filtered through a filter, easily degradable detection targets (e.g., RNA) can be detected with higher sensitivity and accuracy even under non-cooling conditions (e.g., 10 to 35°C, 15 to 30°C, or 15 to 25°C).

Furthermore, when the method of the present invention comprises step c of determining a state of the plant using a value of the signal as an index, the state of the plant can be determined.

The state of the plant includes not only its present state, but also its past and future states. The state of the plant to be determined may include, for example, deficiency in nutrients (e.g., phosphorus, sulfuric acid, and nitrogen), disease infection, flowering time, tuber formation time, dormant bud growth time, the degree of rhizobium symbiosis, and the like.

Specifically, the state can be determined, for example, by comparing the value of the signal with a cutoff value. The cutoff value can be set appropriately by a person skilled in the art from the viewpoints of sensitivity, specificity, positive predictive value, negative predictive value, etc., and can be, for example, a value determined each time or a predetermined value, based on a signal value in a control plant sample, such as a plant sample prepared from a plant cultivated in a medium with sufficient nutrients. The cutoff value can be set, for example, based on a signal value of a control plant sample, or, when there are multiple test samples, the average value, median value, or the like thereof.

Furthermore, the method of the present invention may also be a plant cultivation method that comprises, in addition to step c, step d of adjusting a cultivation condition for the plant based on a result of the determination.

Examples of the adjustment of the cultivation condition include, for example, when it is determined as a result of step c that a nutrient, such as phosphorus, is deficient, applying a fertilizer containing the nutrient or increasing the amount of application; when infection with a disease is determined as a result of step c, suppressing the spread of the disease infection by applying a pesticide, such as a fungicide, partially removing the infected site from the individual, or removing the infected individual from the population; and the like. This allows for more stable cultivation and production of crops, and further improvement in crop productivity.

### Examples

The present invention is described in detail below with reference to Examples. However, the present invention is not limited to these Examples.

### Example 1: Preparation of Amino Group-coated Glass by Liquid Phase Method

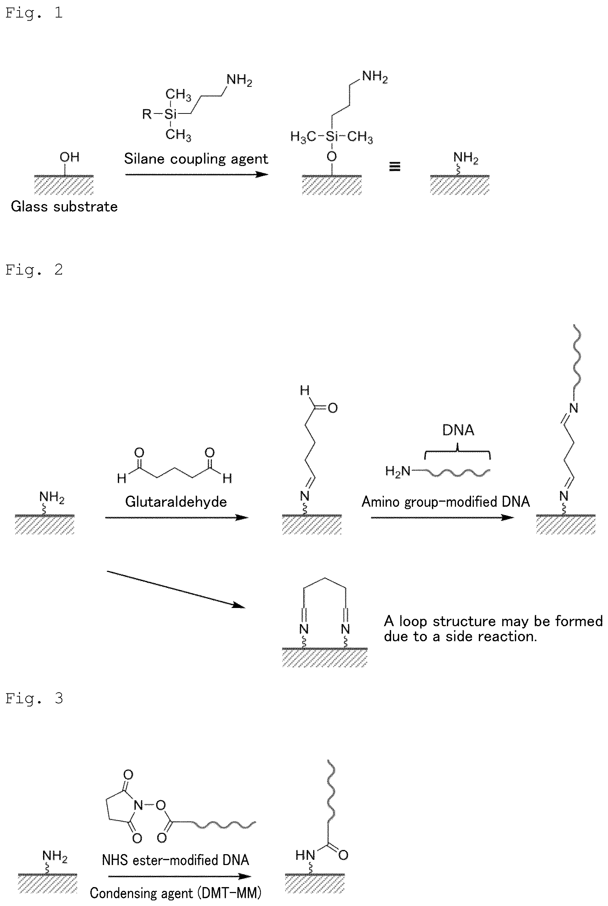

Glass modified with amino groups on its surface was prepared by surface-treating a commercially available glass slide with a silane coupling agent. Fig. 1 shows the reaction scheme. Specifically, the glass was prepared as follows.
(1) The surface of a glass slide was washed with acetone and then subjected to plasma treatment (5 mA, 45 sec).
(2) After plasma treatment, the glass slide was promptly immersed in N,N-dimethylformamide (DMF), a silane coupling agent (3-aminopropyldimethylmethoxysilane (APDMMS) or 3-aminopropyldimethylmethoxysilane (APDMES)) was added to a final concentration of 5%, and triethylamine (TEA) was added to a final concentration of 0.3%.
(3) The reaction liquid in which the glass was immersed was reacted at 80°C for 11 hours or more.
(4) After the reaction was completed, the reaction liquid was cooled to room temperature, and the glass was immersed in chloroform and washed with an ultrasonic cleaner for 10 minutes. The chloroform was exchanged, and the same washing was repeated three times.
(5) The glass was air-dried.

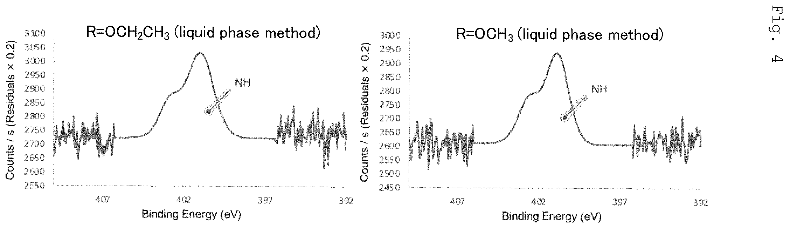

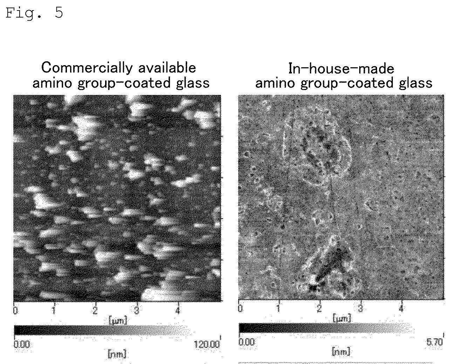

Fig. 4 shows the results of XPS measurement of the surface of the prepared glass. A peak corresponding to an amino group is observed around 401 eV, indicating that the glass surface was modified with amino groups. The results of observing the glass surface with an AFM show that aggregates with a height of about 100 nm and a width of 100 nm were present on the entire surface of commercially available amino group-coated glass (Fig. 5). In contrast, the surface of the in-house-made coated glass prepared using APDMES had aggregates with a height of 10 nm or less and had a flat surface structure (Fig. 5). These results show that the surface structure of the in-house-made coated glass is flatter than that of the commercially available glass, and that the in-house-made coated glass is expected to serve as a substrate that enables more sensitive and stable miRNA detection reactions.

### Example 2: Preparation of Amino Group-coated Glass by Gas Phase Method

To achieve a cheaper and more efficient amino group coating reaction on a glass surface using a silane coupling agent, amino group-coated glass was prepared by a gas phase method. Fig. 1 shows the reaction scheme. Specifically, the glass was prepared as follows.
(1) A piece of filter paper was set on the edge of a glass reaction vessel.
(2) A glass substrate immediately after ozone cleaning was set in the center of the reaction vessel.
(3) A silane coupling agent (3-aminopropyldimethylmethoxysilane (APDMMS) or 3-aminopropyldimethylmethoxysilane (APDMES)) was added dropwise onto the piece of filter paper in an amount of 0.625 µl per cm² of the glass surface area.
(4) A lid was quickly placed on the reaction vessel, and the reaction vessel was allowed to stand at room temperature overnight.
(5) After the reaction was completed, the glass was immersed in chloroform and washed with an ultrasonic cleaner for 10 minutes. The chloroform was exchanged, and the same washing was repeated three times.
(6) The glass was air-dried.

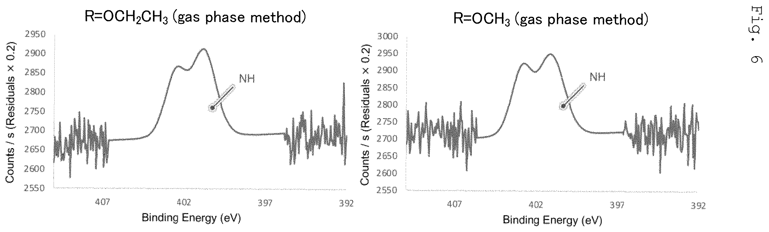

The results of XPS analysis of the glass whose surface was coated by the gas phase method show that a peak corresponding to an amino group was observed around 401 eV as in the liquid phase method (Fig. 6). The results show that amino-coated glass was also prepared by the gas-phase reaction.

### Example 3: Immobilization of DNA Using NHS Ester and Placement of Detection Device

To confirm the immobilization of a DNA probe (Fig. 2 or 3) and the quality of amino group-coated glass, a PDMS device (linear PDMS device) with a flow channel (flow channel width: 500 µm, flow channel height: 100 µm) having a liquid inlet port and a liquid suction port was used. A liquid was introduced into and passed through the flow channel by introducing the liquid into the inlet port using a pipette. The details are as follows.
(1) The PDMS device for DNA probe immobilization was placed on a glass slide in which amino groups were introduced (commercially available glass or in-house-made glass).
(2) A 2 µM NHS group-modified DNA probe (NHS-DNA-miR399: NHS-TTTTTTTTTTTTTTTCAGGGCAACT (SEQ ID NO: 1)) and 50 mM 4-(4,6-dimethoxy-1,3,5-triazin-2-yl)-4-methylmorpholinium chloride (DMT-MM) were prepared in a 100 mM MOPS buffer (pH of 7.0), and incubation was performed at 25°C overnight.
(3) The glass surface was washed by introducing a wash buffer (0.5×SSC, 0.1% SDS) into the PDMS device for DNA probe immobilization. The glass after washing was further washed by introducing ultrapure water twice.
(4) A blocking solution (1×blocking solution (DIG Wash and Block Buffer Set, Roche)) was introduced into the flow channel, and incubation was performed at room temperature for 60 minutes.
(5) A sample solution (composition: miR399 (UGCCAAAGGAGAGUUGCCCUG (SEQ ID NO: 2)) (0 nM or 1 nM), a biotinylated DNA probe (CTCCTTTGGCA (SEQ ID NO: 3)-[biotin]) (0.4 µM), and a buffer solution (1×TE, 1×PBS), and dithiothreitol (1 mM)) were prepared in a tube.
(6) 10 µl of the sample solution was introduced into the flow channel of a detection device, and hybridization was performed for 30 minutes.
(7) 20 µl of Streptavidin-Alexa (Streptavidin Alexa Fluor 555 conjugate, Thermo Fisher Scientific) (500×) was introduced into the flow channel, and incubation was performed at room temperature for 15 minutes.
(8) 40 µl of a wash buffer (1×wash buffer (DIG Wash and Block Buffer Set, Roche)) was introduced into the flow channel for washing.
(9) 20 µl of a biotinylated anti-streptavidin antibody (7.5 µg/ml) was introduced into the flow channel, and incubation was performed at room temperature for 10 minutes.
(10) 20 µl of Streptavidin-Alexa (Streptavidin Alexa Fluor 555 conjugate, Thermo Fisher Scientific) (500×) was introduced into the flow channel, and incubation was performed at room temperature for 10 minutes.
(11) 40 µl of a wash buffer (1×wash buffer (DIG Wash and Block Buffer Set, Roche)) was introduced to wash the flow channel. A fluorescence image of the flow channel chip was obtained using a fluorescence microscope.
(13) The amount of fluorescence was quantified from the fluorescence image (image analysis software ImageJ, National Institutes of Health).

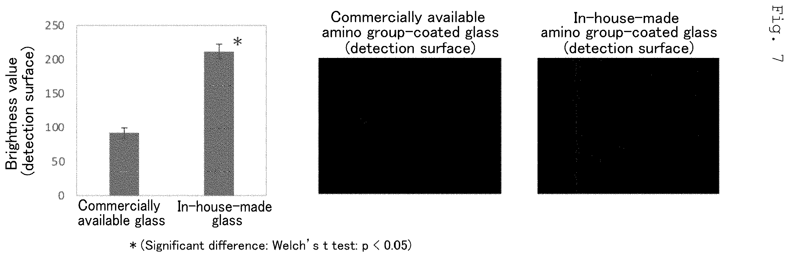

Fig. 7 shows the results of comparing the detection signal of 1 nM miR399 between the commercially available amino group-coated glass and the in-house-made amino group-coated glass. A comparison of the detection amounts between the commercially available glass and the in-house-made glass reveals that the brightness value was 91.9 when the commercially available glass was used, whereas the brightness value was 211.4 when the in-house-made glass was used, indicating a significant increase in the detection amount (Welch's t test: p < 0.05). This result shows that the use of the in-house-made glass as the glass substrate provides higher detection sensitivity.

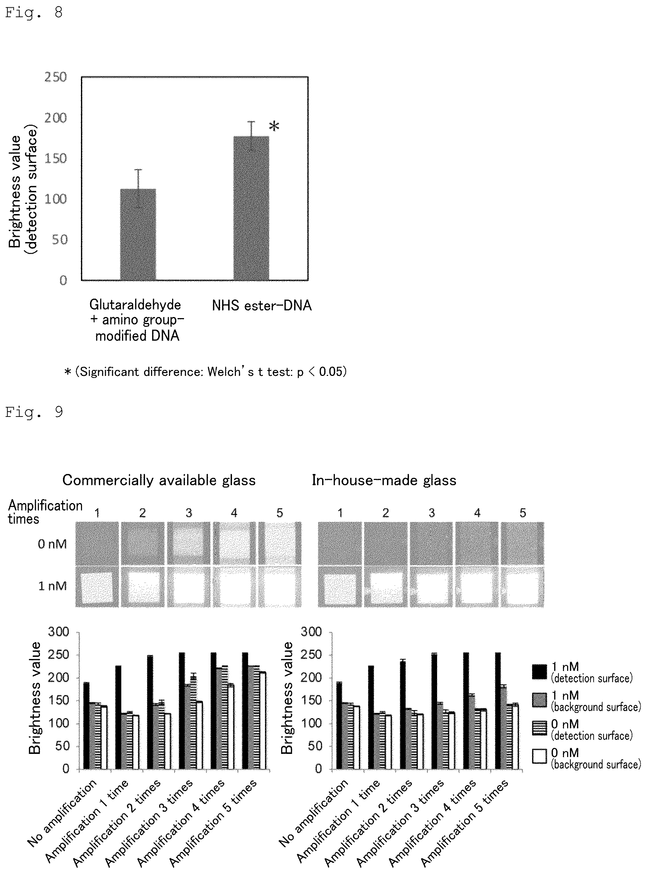

Fig. 8 shows the results of comparing a conventional method and a method using an NHS ester as a method for immobilizing DNA, using the in-house-made amino group-coated glass. The detection amount of miR399 was 112.8 when the conventional method was used, whereas, by using the NHS ester, the detection amount was significantly increased to 177.5 (Welch's t test: p < 0.05). This result shows that the simplification of the method for immobilizing a DNA probe and the enhancement of detection sensitivity were successfully achieved.

### Example 4: Detection of Signal on Detection Surface When Signal Amplification Is Performed

Using a DNA probe immobilization method that uses an in-house-made amino group-coated glass and NHS ester-DNA, a signal amplification experiment using a biotinylated anti-streptavidin antibody was performed. A DNA probe was immobilized on the glass slide in which amino groups were introduced using a PDMS device with a flow channel (flow channel width: 500 µm, flow channel height: 20 µm) having a liquid inlet port and a liquid suction port (a PDMS device for DNA probe immobilization). A diagnostic device was prepared by placing a PDMS device with six liquid inlet ports, six liquid suction ports, and six flow channels (flow channel width: 500 µm, flow channel height: 20 µm) connecting these ports (a detection PDMS device) on the glass slide having the DNA immobilized thereon. Detection of artificial miR399 and signal amplification were performed using the prepared detection device. A liquid was introduced into and passed through the flow channel by introducing the liquid into the inlet ports with a pipette and sucking the liquid from the suction ports with a syringe pump. The details are as follows.
(1) 1 nM miR399 (UGCCAAAGGAGAGUUGCCCUG (SEQ ID NO: 2)) was mixed with a solution for a sample (biotinylated DNA probe (CTCCTTTGGCA (SEQ ID NO: 3)-[biotin]) (0.4 µM), a buffer solution (1×TE, 1×PBS), and dithiothreitol (1 mM)) (5 µl).
(2) 10 µl of the resulting sample solution was introduced into the flow channels of the detection device, and hybridization was performed (pump: 0.3 µl/min, 30 minutes).
(3) Streptavidin-Alexa (Streptavidin Alexa Fluor 555 conjugate, Thermo Fisher Scientific) (500×) was introduced into the flow channels (pump: 1.5 µl/min, 15 minutes).
(4) The flow channels were washed with a wash buffer (1×wash buffer (DIG Wash and Block Buffer Set, Roche)) (pump: 0.3 µl/min, 15 minutes).
(5) A biotinylated anti-streptavidin antibody (7.5 µg/ml) was introduced into the flow channels (pump: 1.5 µl/min, 10 minutes).
(6) Streptavidin-Alexa (Streptavidin Alexa Fluor 555 conjugate, Thermo Fisher Scientific) (500×) was introduced into the flow channels (pump: 1.5 µl/min, 10 minutes).
(7) The flow channels were washed with a wash buffer (1×wash buffer (DIG Wash and Block Buffer Set, Roche)) (pump: 0.3 µl/min, 10 minutes).
(8) The introduction of the biotinylated anti-streptavidin antibody, Streptavidin-Alexa, and wash buffer described above was performed five times to perform signal amplification.
(9) A fluorescence image of the flow channel chip was obtained using a fluorescence microscope.
(10) The amount of fluorescence was quantified from the fluorescence image (image analysis software ImageJ, National Institutes of Health).

Fig. 9 shows the results. 1 nM miR399 was detected in both the commercially available glass and the in-house-made glass, with significant difference (Welch's t test: p < 0.05), compared with the blank (0 nM) sample values. On the other hand, as a result of performing signal amplification on the sample without miR399, the in-house-made glass showed a limited increase in signal on the detection surface due to the signal amplification process, whereas the commercially available glass showed strong nonspecific signals on the detection surface. This result shows that the in-house-made amino group-coated glass exhibits less nonspecific signal detection and enables accurate detection of miR399.

### Example 5: Detection of Stress Factor in Tomato Using Diagnostic Technique

miR399 was detected from tomato samples using a detection device. A DNA probe was immobilized on a glass slide in which amino groups were introduced, using a PDMS device with 10 flow channels (flow channel width: 200 µm, flow channel height: 20 µm) each having a liquid inlet port and a liquid suction port. A diagnostic device was prepared by placing a PDMS device with 12 liquid inlet ports, 12 liquid suction ports, and 12 flow channels (flow channel width: 500 µm, flow channel height: 20 µm) connecting these ports on the glass slide with the DNA immobilized thereon. A liquid was introduced into and passed through the flow channels by introducing the liquid into the inlet ports with a pipette and sucking the liquid from the suction ports with a syringe pump. The details are as follows.
(1) To true leaves of a tomato, an equal weight of a 100 mM DTT/90 mM Tris-HCl (pH of 7.6) buffer was added, and the leaves were ground with a pestle.
(2) The ground tomato liquid was centrifuged at 14000 rpm and 4°C for 10 minutes, and the supernatant was collected.
(3) The tomato supernatant was roughly purified by passing it through a filter (Nanosep; MWCO: 30K).
(4) A sample solution (5 µl of the roughly purified tomato sample, a biotinylated DNA probe (CTCCTTTGGCA (SEQ ID NO: 3)-[biotin]) (0.4 µM), a buffer solution (1×TE, 1×PBS), and dithiothreitol (1 mM)) was prepared in a tube.
(5) 10 µl of the sample solution was introduced into the flow channels of the detection device, and hybridization was performed (pump: 0.3 µl/min, 30 minutes).
(6) Streptavidin-Alexa (Streptavidin Alexa Fluor 555 conjugate, Thermo Fisher Scientific) (500×) was introduced into the flow channels (pump: 1.5 µl/min, 15 minutes).
(7) The flow channels were washed with a wash buffer (1×wash buffer (DIG Wash and Block Buffer Set, Roche)) (pump: 0.3 µl/min, 15 minutes).
(8) A biotinylated anti-streptavidin antibody (7.5 µg/ml) was introduced into the flow channels (pump: 1.5 µl/min, 10 minutes).
(9) Streptavidin-Alexa (Streptavidin Alexa Fluor 555 conjugate, Thermo Fisher Scientific) (500×) was introduced into the flow channels (pump: 1.5 µl/min, 10 minutes).
(10) The flow channels were washed with a wash buffer (1×wash buffer (DIG Wash and Block Buffer Set, Roche)) (pump: 0.3 µl/min, 10 minutes).
(11) The introduction of the biotinylated anti-streptavidin antibody, Streptavidin-Alexa, and wash buffer described above was performed five times to perform signal amplification.
   A fluorescence image of the flow channel chip was obtained with a fluorescence microscope.
(13) The amount of fluorescence was quantified from the fluorescence image (image analysis software ImageJ, National Institutes of Health).

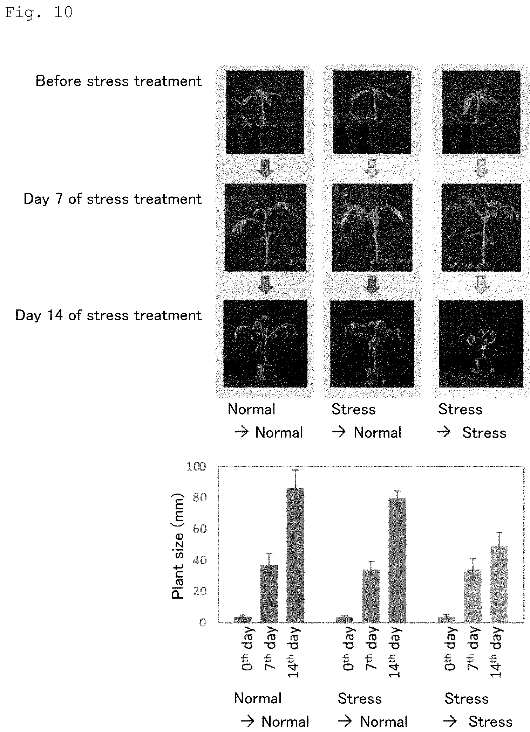

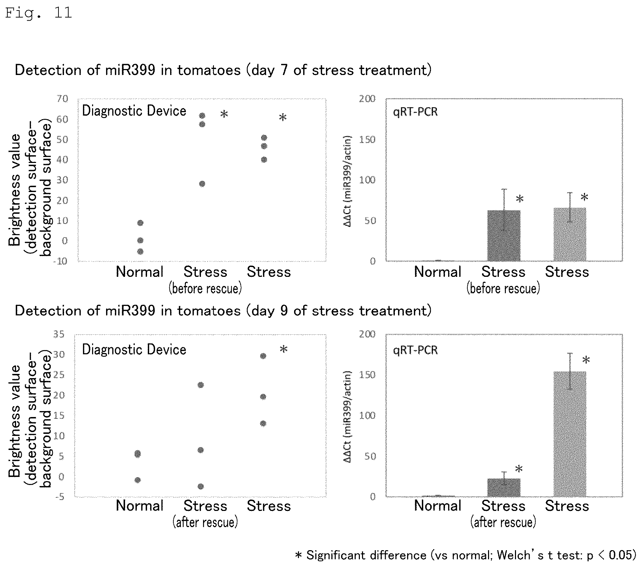

Fig. 10 shows the growth states of tomatoes subjected to stress treatment. There was no difference in plant size between tomatoes subjected to stress treatment for 7 days and a normally grown tomato, and it was difficult to determine the stress state from their appearance. Fig. 11 shows the results of detecting miR399 in these tomatoes using the device. The tomato samples subjected to stress treatment for 7 days showed significantly higher brightness values than the tomato normally grown for 7 days (Welch's t test: p < 0.05). This result shows that miR399, which was highly expressed due to stress treatment, was detected with the diagnostic device. Similar results were shown in qRT-PCR, which is used for detecting miRNA. A tomato diagnosed with stress was rescued from the stressful environment by switching to normal growing conditions. The result of a diagnosis made on the tomato grown for 2 days after the rescue treatment reveals that the tomato showed no significant difference in brightness compared with that of normal treatment. However, a tomato that continued to receive stress treatment showed a significantly high brightness value (Welch's t test: p < 0.05). This result shows that the cessation of stress response due to rescue from stress treatment was detected with the diagnostic device. The decrease in the expression level of miR399 by rescue was also shown in qRT-PCR. The tomato rescued from stress treatment after diagnosis had a plant size after 14 days that was similar to that of the normally grown tomato, whereas the tomato that continued to receive stress treatment for 14 days showed growth inhibition due to stress damage (Fig. 10). Thus, this technique can be used as a simple diagnostic device for cultivation management to avoid stress damage by detecting miR399, which is a stress factor.

### Example 6: Simultaneous Diagnosis of Multiple Items Using Multiple Lanes

To verify whether miRNAs other than miR399 can be detected using the technique of the present invention, miRNA detection from tomato samples was performed using NHS-DNA probes having sequences different from that of miR399. When immobilizing the DNA probes, a PDMS device with a flow channel (flow channel width: 200 µm, flow channel height: 20 µm) having a liquid inlet port and a liquid suction port was used to simultaneously perform a diagnosis on other targets. A diagnostic device was prepared by placing a PDMS device with a liquid inlet port, a liquid suction port, and a flow channel (flow channel width: 200 µm, flow channel height: 20 µm) connecting these ports on a glass slide having the DNAs immobilized thereon.
(1) A 10-lane PDMS device for DNA probe immobilization was placed on a glass slide in which amino groups were introduced.
(2) 2 µM NHS group-modified DNA probes (NHS-DNA-miR395: NHS-TTTTTTTTTTTTTTTGAGTCCCCC (SEQ ID NO: 4), NHS-DNA-miR164: NHS-TTTTTTTTTTTTTTTTGCACGTGCC (SEQ ID NO: 5), NHS-DNA-miR319: NHS-TTTTTTTTTTTTTTTAGGGAGCTCC (SEQ ID NO: 6), NHS-DNA-miR171: NHS-TTTTTTTTTTTTTTTTGATATTGGCG (SEQ ID NO: 7), NHS-DNA-miR168: NHS-TTTTTTTTTTTTTTTTTTCCCGACCT(SEQ ID NO: 8)) and 50 mM 4-(4,6-dimethoxy-1,3,5-triazin-2-yl)-4-methylmorpholinium chloride (DMT-MM) were prepared in 100 mM MOPS buffer (pH of 7.0), and incubation was performed at 25°C overnight. At this time, the regions on the glass slide where the DNA probes were immobilized were marked with a marker from the back of the glass slide.
(3) The PDMS device for DNA probe immobilization was removed with the device immersed in a wash buffer (0.5×SSC, 0.1% SDS), and the glass surface was washed. The glass after washing was further washed twice with ultrapure water, and excess water was blown off with a blower.
(4) The PDMS device for detection was placed on the glass slide having the DNA probes immobilized thereon, followed by degassing for 15 minutes. In this step, the PDMS device for detection was placed so that the lines on which the DNA probes were immobilized and the flow channel of the PDMS device for detection were perpendicular to each other, based on the marks made before removal.
(5) A blocking solution (1×blocking solution (DIG Wash and Block Buffer Set, Roche)) was introduced into the flow channel, and incubation was performed at room temperature for 60 minutes.
(6) To true leaves of a tomato, an equal weight of a 100 mM DTT/90 mM Tris-HCl (pH of 7.6) buffer was added, and the leaves were ground with a pestle.
(7) The ground tomato liquid was centrifuged at 14000 rpm and 4°C for 10 minutes, and the supernatant was collected.
(8) The tomato supernatant was roughly purified by passing it through a filter (Nanosep; MWCO: 30K).
(9) A sample solution (5 µl of the roughly purified tomato sample, a biotinylated DNA probe (CTCCTTTGGCA (SEQ ID NO: 3)-[biotin]) (0.4 µM), a buffer solution (1×TE, 1×PBS), and dithiothreitol (1 mM)) was prepared in a tube.
(10) 10 µl of the sample solution was introduced into the flow channel of the detection device, and hybridization was performed (pump: 0.3 µl/min, 30 minutes).
(11) Streptavidin-Alexa (Streptavidin Alexa Fluor 555 conjugate, Thermo Fisher Scientific) (500×) was introduced into the flow channel (pump: 1.5 µl/min, 15 minutes).
(12) The flow channel was washed with a wash buffer (1×wash buffer (DIG Wash and Block Buffer Set, Roche)) (pump: 0.3 µl/min, 15 minutes).
(13) A biotinylated anti-streptavidin antibody (7.5 µg/ml) was introduced into the flow channel (pump: 1.5 µl/min, 10 minutes).
(14) Streptavidin-Alexa (Streptavidin Alexa Fluor 555 conjugate, Thermo Fisher Scientific) (500×) was introduced into the flow channel (pump: 1.5 µl/min, 10 minutes).
(15) The flow channel was washed with a wash buffer (1×wash buffer (DIG Wash and Block Buffer Set, Roche)) (pump: 0.3 µl/min, 10 minutes).
(16) The introduction of the biotinylated anti-streptavidin antibody, Streptavidin-Alexa, and wash buffer described above was performed five times to perform signal amplification.
   A fluorescence image of the flow channel chip was obtained with a fluorescence microscope.
(18) The amount of fluorescence was quantified from the fluorescence image (image analysis software ImageJ, National Institutes of Health).

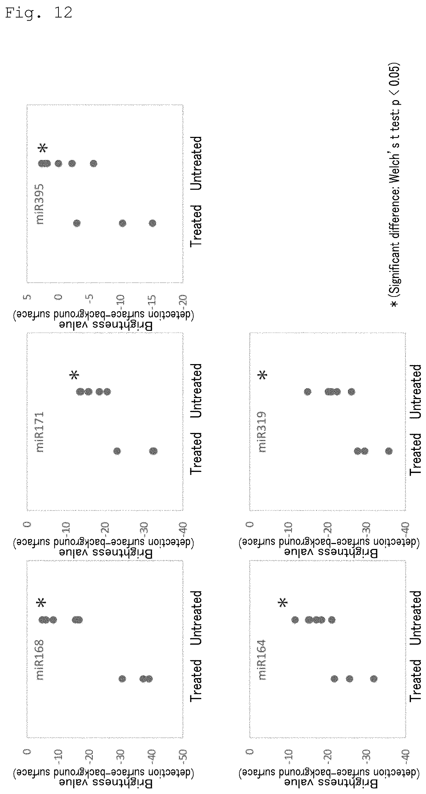

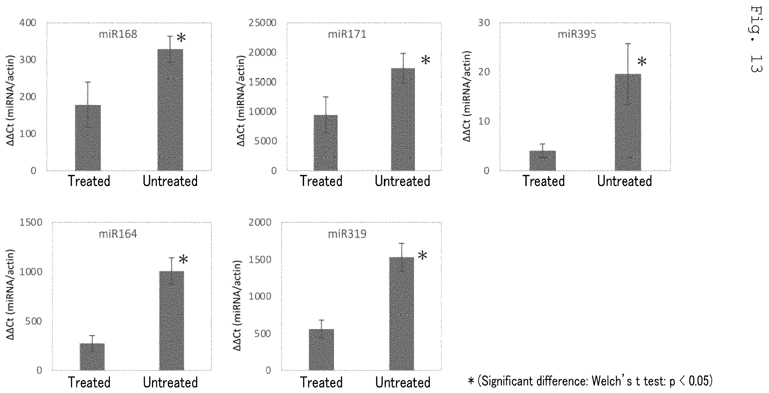

Fig. 12 shows the results of signal detection of the tomato samples treated to express the respective miRNAs, using the diagnostic device. Compared with an untreated tomato, the tomatoes subjected to stress treatment showed significant increases in signal (Welch's t test: p < 0.05). In fact, when the expression levels of these target miRNAs were examined by qRT-PCR, the expression levels of all of the target miRNAs significantly increased (Welch's t test: p < 0.05) (Fig. 13). These results show that this technique is also capable of detecting targets other than miR399.

## Claims

1. A nucleic acid detection chip comprising an inorganic substrate and a nucleic acid probe,
(x) the nucleic acid probe being linked via an amino group linked to the inorganic substrate; and/or
(y) the nucleic acid probe being linked by an amide bond between an amino group linked to the inorganic substrate and the nucleic acid probe that has an active ester group or a carboxy group.

2. The nucleic acid detection chip according to claim 1,
wherein
(x1) a group represented by formula (x1): 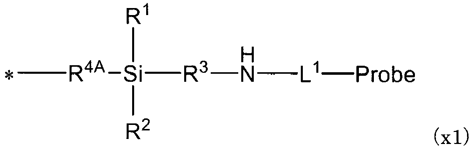
wherein R¹ and R² are the same or different, and each represents an alkyl group, a hydrogen atom, an alkoxy group, a halogen atom, a site of attachment to a silane coupling agent, or a site of attachment to the inorganic substrate; R³ represents a linker; R^{4A} represents an oxygen atom or a single bond; L¹ represents a linker; Probe represents a nucleic acid probe; and * represents a site of attachment to the inorganic substrate,
is attached to the inorganic substrate; and/or
(y1) a group represented by formula (y1): 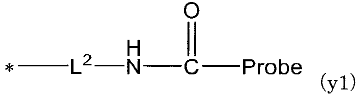
wherein L² represents a linker; Probe represents a nucleic acid probe; and * represents a site of attachment to the inorganic substrate,
is attached to the inorganic substrate.

3. The nucleic acid detection chip according to claim 1, wherein (xy) the nucleic acid probe is linked by an amide bond between an amino group linked to the inorganic substrate and the nucleic acid probe that has an active ester group or a carboxy group.

4. The nucleic acid detection chip according to claim 3, wherein (xy1) a group represented by formula (xy1): 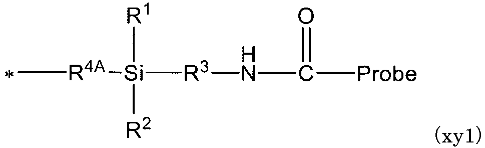
wherein R¹ and R² are the same or different, and each represents an alkyl group, a hydrogen atom, an alkoxy group, a halogen atom, a site of attachment to a silane coupling agent, or a site of attachment to the inorganic substrate; R³ represents a linker; R^{4A} represents an oxygen atom or a single bond; Probe represents a nucleic acid probe; and * represents a site of attachment to the inorganic substrate,
is attached to the inorganic substrate.

5. The nucleic acid detection chip according to claim 4, wherein R¹ and R² are the same or different, and each represents an alkyl group.

6. The nucleic acid detection chip according to claim 1, wherein the nucleic acid probe is an mRNA and/or miRNA detection probe.

7. The nucleic acid detection chip according to claim 1, comprising:
(A) a sample injection portion; and
(C) a flow channel connected to the sample injection portion A and having a nucleic acid probe immobilization region.

8. The nucleic acid detection chip according to claim 1, which is for use in detecting a plant nucleic acid.

9. A method for determining a state of a plant, comprising:
(a) bringing a sample of the plant into contact with the nucleic acid detection chip of any one of claims 1 to 8;
(b) detecting a signal on the nucleic acid probe; and
(c) determining the state of the plant using a value of the signal as an index.

10. A method for cultivating a plant, comprising:
(a) bringing a sample of the plant into contact with the nucleic acid detection chip of any one of claims 1 to 8;
(b) detecting a signal on the nucleic acid probe;
(c) determining a state of the plant using a value of the signal as an index; and
(d) adjusting a cultivation condition for the plant based on a result of the determination.

11. A method for producing the nucleic acid detection chip of any one of claims 1 to 8, comprising:
(X) reacting an inorganic substrate with a silane coupling agent; and/or
(Y) reacting an amino group linked to the inorganic substrate with a nucleic acid probe having an active ester group or a carboxy group.
